# EUROPÄISCHE PATENTSCHRIFT

(11) **EP 0 774 954 B1**
(45) Veröffentlichungstag und Bekanntmachung des Hinweises auf die Patenterteilung: **04.08.1999**
(21) Anmeldenummer: 95924231.4
(22) Anmeldetag: 14.06.1995
(51) Int. Cl.: A61K 7/48, A61K 9/70

(54) **HYDROGEL FÜR DIE APPLIKATION VON THERAPEUTISCHEN UND/ODER KOSMETISCHEN WIRKSTOFFEN AN DIE HAUT**
HYDROGEL FOR THE APPLICATION OF THERAPEUTIC AND/OR COSMETIC ACTIVE SUBSTANCES TO THE SKIN
HYDROGEL POUR APPLICATION DE PRINCIPES ACTIFS THERAPEUTIQUES ET/OU COSMETIQUES SUR LA PEAU

(30) Priorität: 05.07.1994 DE 4423457; 23.12.1994 DE 4446380
(43) Veröffentlichungstag der Anmeldung: 28.05.1997
(73) Patentinhaber: LTS LOHMANN Therapie-Systeme GmbH, 56567 Neuwied (DE)
(72) Erfinder: BERGER, Silke, D-56305 Puderbach (DE); VON KLEINSORGEN, Reinhard, D-56170 Bendorf (DE); SIMON, Günter, D-54576 Hillesheim (DE)
(74) Vertreter: Flaccus, Rolf-Dieter, Dr.
(86) Internationale Anmeldenummer: EP9502302
(87) Internationale Veröffentlichungsnummer: WO9601100

(56) Entgegenhaltungen:
- EP-A- 0 309 309
- EP-A- 0 446 636
- EP-A- 0 461 085
- WO-A-92/02204
- WO-A-92/15289
- FR-A- 2 538 247
- GB-A- 2 280 906
- US-A- 3 499 446
- US-A- 4 973 466

## Beschreibung

Die Erfindung betrifft ein Hydrogel als Applikationsform für den Einsatz von wirkstoffen zur Therapierung von durch Verletzungen, UV-Strahlen, Alterung oder anderweitig verursachten Hautschäden oder von empfindlichen Haut- und Nagelstellen.

Beispielsweise verursacht Hautalterung eine sinkende Hautelastizität. Diese wiederum ist maßgeblich auf ein Verkleben einzelner Kollagenfasern innerhalb der netzartigen Struktur der Dermis zurückzuführen. Mit sinkender Hautelastizität geht ein reduziertes Wasserbindevermögen der Epidermis einher. Zur Erhaltung oder Wiederherstellung der natürlichen Hautfunktion ist daher eine Feuchtigkeitszufuhr von außen unerläßlich. Zusätzlich kann das Hautrelief durch Einwirkung von hautglättenden, pflegenden und anregenden Wirkstoffen positiv beeinflußt und die Durchblutung angeregt werden. Andere Hautschäden wie z.B. Sonnenbrand, Entzündungen etc. machen den Einsatz heilender und/oder kosmetischer Wirkstoffe auf der Oberfläche der Haut erforderlich.

Zur Erfüllung dieser Erfordernisse sind verschiedene Applikationsformen für Therapeutika bekannt. Beispielsweise können Emulsionen, die Mehr-Phasen-Systeme darstellen, in Form von Cremes oder Lotionen auf die Haut aufgetragen werden. Ebenfalls können Öle, die einphasige, nur lipidlösliche Rohstoffe enthaltende Syteme sind, in flüssigem Zustand auf die Haut aufgetragen werden. Weiterhin sind als Heil- und/oder Pflegesubstanzen halbfeste, transparente Gele bekannt, die cremeartige Konsistenz haben.

Die Anwendung solcher Heil- und Pflegesubstanzen auf der Hautoberfläche bereitet in der Praxis oftmals erhebliche Schwierigkeiten. Zuviel aufgetragene Substanz kann von der Haut nicht oder nur sehr langsam aufgenommen werden. So können z.B. bei der Antifaltenbehandlung im Bereich der empfindlichen Unteraugenpartie Substanzen mit der Augenschleimhaut in Berührung kommen und dort Irritationen auslösen.

Eine bekannte Möglichkeit, das Applikations- und Dosierproblem zu überwinden, ist die Anwendung von getränkten Vliespads. Diese sind durch den nassen, instabilen Aufbau nur schwer und unsauber auf die vorgesehene Hautpartie zu applizieren.

Die Verwendung starrer sowie flächenförmiger Hydrogele als Träger von Wirkstoffen für deren Applikation zur Behandlung der Haut bzw. in der Kosmetik ist bisher nicht bekannt. Starre Gelzubereitungen werden bisher nur als Dosierform für aktive chemische Verbindungen eingesetzt. Als Dosierform sind zwei unterschiedliche Arten von Gelatinekapseln bekannt: harte und weiche Kapseln (DE-OS 2316 242). Bekannt ist weiterhin ein aus Gel bestehendes Applikationssystem (CA 2.078.960).

Außerdem werden starre Gelzubereitungen in der Chromatographie verwendet (US 5,277,915).

Der Erfindung liegt die Aufgabe zugrunde, Schwierigkeiten bei der Applikation von Wirkstoffen für die kosmetische Pflege und/oder Behandlung von Hautschäden bzw. von empfindlichen Haut- und Nagelstellen zu vermindern, die Anwendung von Therapeutika zu vereinfachen und zur Heilung und Pflege der Haut bzw. zur Erhaltung der natürlichen Hautfunktion beizutragen.

Diese Aufgabe wird erfindungsgemäß dadurch gelöst, daß als Applikationsform für den Einsatz von wirkstoffen zur Therapierung der Haut Hydrogele zur Behandlung von Hautschäden sowie zur Anwendung in der Kosmetik verwendet werden, die flächenförmig ausgebildete und an Konturen menschlicher Körperstellen angepaßte starr-elastische Gebilde sind und geeignete Wirkstoffe zur Behandlung der Haut enthalten.

Um sich an Konturen von Stellen des menschlichen Körpers anzupassen, sind Hydrogele entsprechend dimensioniert. Für die Anwendung gegen Falten unter den Augen sind sie z.B. nierenförmig ausgebildet. Damit decken sie auch die Randbereiche der Augen ab, wo die "Krähenfüße" (stärkere Falten) entstehen.

Als kühlende Augenmasken sind die Hydrogele rund, z.B. wie Gurkenscheiben ausgebildet. Diese werden auf die geschlossenen Augen gelegt.

Die mit der Erfindung erzielten Vorteile bestehen insbesondre darin, daß statt einer halbfesten oder flüssigen Zubereitung ein starres, elastisches Hydrogel verwendet wird, welches eine saubere Applikation ermöglicht.

Durch die Ausbildung mit einer sanft klebenden Oberfläche haften die Hydrogele auf der Haut, so daß die Bewegungsfreiheit während der Behandlung nicht völlig eingeschränkt wird. Nach der Anwendung können die Hydrogele schmerzlos und rückstandsfrei abgezogen werden.

Die Hydrogele können mit bekannten Techniken hergestellt werden. Ein einfaches Verfahren ist z.B. das Dispergieren der Ausgangsstoffe in erhitztem Wasser und Gelieren der Lösung bei anschließender Kühlung. Die erhitzte Lösung kann mit Hilfe eines Dosierautomaten in ein Tiefziehteil eingefüllt werden. Dabei können flächige Hydrogele mit einer Dicke von 0,5 - 10 mm hergstellt werden, wobei je nach Verwendungszweck überwiegend 1 - 3 mm dicke Gebilde von Hydrogelen besonders vorteilhaft sind.

Durch den Einsatz verschiedener heilender, z.B. entzündungshemmender und/oder pflegender Wirkstoffe aus den Gruppen von Acetylsalicylsäure (ASS), der Vitamine, Feuchthaltemittel, pflanzlichen oder ätherischen Öle - um nur einige zu nennen - ist das Anwendungsgebiet der erfindungsgemäßen Hydrogele breit gefächert. Ein Anwendungsbeispiel ist die Behandlung der Haut bei vorzeitiger Alterung und Faltenbildung, die z.B. durch extensive Sonnenlicht-Exposition der Haut entsteht und mit Hydrogelen, die Vitamin E enthalten, behandelt wird. Untersuchungen haben gezeigt, daß RRR-Tocopherole als Radikalfänger von reaktiven Verbindungen agieren, die nach Einwirkung von UV-Strahlen entstehen. Ein anderes Anwendungsbeispiel ist eine Augenmaske, die die Augen völlig abdeckt. Die bevorzugt transparente Maske wirkt durch ihre intensiv kühlende Eigenschaft belebend und erfrischend und gibt pflegende Substanzen wie z.B. Panthenol an die Haut ab.

Im folgenden wird ein Ausführungsbeispiel der Erfindung beschrieben:

Die starre Hydrogelbildung wird erreicht durch die Zusammensetzung, enthaltend:

| Hydrogel zu Antifaltenbildung | |
|---|---|
| a) Komponenten: | Gew.-% |
| Wasser | 34,12 |
| Glycerin | 26,66 |
| Gelatine A (Bloom-Wert 200) | 15,46 |
| Saccharose | 10,66 |
| Dexpanthenol | 8,00 |
| Polyvinylpyrrolidon | 2,67 |
| Feuchthaltefaktor | 2,13 |
| Konservierungsmittel | 0,30 |

| b) Herstellung: | |
|---|---|
| Polyvinylpyrrolidon wird unter Rühren in Wasser gelöst. Sodann wird Konservierungsmittel und Glycerin dazugegeben. Gelatine A wird ca. 10 min in dem vorgelegten Wasser quellen gelassen und die Mischung auf ca. 65°C erhitzt und bis zur klaren Lösung gerührt. Schließlich werden Saccharose, Dexpanthenol und kosmetische Wirkstoffe dazugegeben. | |

Ein Anwendungsbeispiel der Erfindung ist in den Zeichnungen dargestellt und wird im folgenden näher beschrieben: Es zeigen:

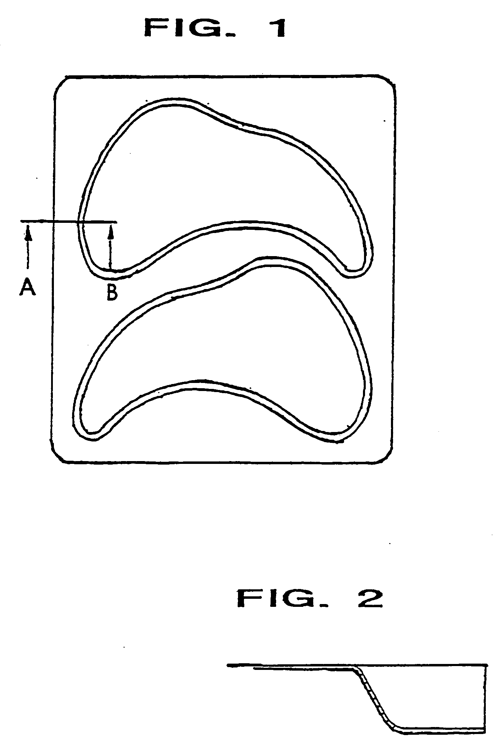

- FIG.1: In Draufsicht ein Tiefziehteil für Hydrogele zur Antifaltenbehandlung. Darin befinden sich zwei Hydrogel-Patches zur Einmalverwendung unter den Augen;
- FIG.2: einen Querschnitt A-B aus FIG.1, der die Wandung und Höhe des Tiefziehteils zeigt.

Die Erfindung ist nicht nur auf die Behandlung von alterungsbedingten Schäden der Haut beschränkt.

Vielmehr sollen die wirkstoffhaltigen Hydrogele für alle Arten äußerer Heil- und Pflegebehandlung eingesetzt werden, z.B. anstelle von Pflastern, die eine Behandlung unter zeitweiser oder längerdauernder Therapierung bestimmter Hautstellen erforderlich machen.

Darüberhinaus eignen sich die Hydrogele zur Abgabe von Wirkstoffen an Pflanzen.

## Patentansprüche

1. Hydrogel für die Applikation von therapeutischen und/oder kosmetischen Wirkstoffen an die Haut bzw. für die kosmetische Pflege und Behandlung empfindlicher Haut- und Nagelstellen, dadurch gekennzeichnet, daß es ein flächenförmig ausgebildetes und an Konturen menschlicher Körperstellen angepaßtes starr-elastisches Gebilde ist, welches medizinische und/oder kosmetische Wirkstoffe enthält.

2. Hydrogel nach Anspruch 1, dadurch gekennzeichnet, daß es mit einer haftklebenden Oberfläche ausgebildet ist.

3. Hydrogel nach Anspruch 1 oder 2, dadurch gekennzeichnet, daß darin enthaltene Wirkstoffe Therapeutika zur Heilung von durch Verletzungen, UV-Bestrahlung, Alterung oder anderweitig verursachter Hautschäden sind.

4. Hydrogel nach Anspruch 1 bis 3, dadurch gekennzeichnet, daß darin enthaltene Wirkstoffe hautglättend sind und aus der Gruppe, bestehend aus Vitaminen, Feuchthaltemitteln und pflanzlichen Ölen ausgewählt sind.

5. Hydrogele nach Anspruch 1 - 4, dadurch gekennzeichnet, daß die darin enthaltenen Wirkstoffe hautfunktionsanregend sind und aus der Gruppe, bestehend aus Pflanzenextrakten und ätherischen Ölen ausgewählt sind.

6. Hydrogel nach einem oder mehreren der Ansprüche 1 bis 5, dadurch gekennzeichnet, daß es der von der Haut abgewandten Seite mit einer nichtklebenden Schicht ausgerüstet ist.

7. Hydrogel nach einem oder mehreren der Ansprüche 1 bis 6, dadurch gekennzeichnet, daß es vor der Applikation in einer wasser- und wasserdampfdichten Verpackung verpackt ist.

8. Verwendung des Hydrogels nach einem der Ansprüche 1 bis 7 zur Herstellung eines Mittels zur Heilung von Hautschäden.

9. Verwendung des Hydrogels nach einem der Ansprüche 1 bis 7 zur kosmetischen Hautpflege, insbesondere Hautglättung bzw. Hautverjüngung.

10. Verwendung des Hydrogels nach einem der Ansprüche 1 bis 7 zur Herstellung eines Mittels zur Beeinflußung der Durchblutung der Haut.

11. Verwendung des Hydrogels nach den Ansprüchen 1 bis 7 zur Herstellung eines Mittels zur Nagelpflege.

12. Verwendung des Hydrogels nach den Ansprüchen 1 bis 7 zur Abgabe von Wirkstoffen an Pflanzen.

## Claims

1. A hydrogel for the application of therapeutic and/or cosmetic active substances to the skin or for the cosmetic care and treatment of sensitive sites of the skin and nails, characterized in that it is a sheet-like, rigid-elastic structure adapted to contours of human body sites which comprises medicinal and/or cosmetic active substances.

2. The hydrogel according to claim 1 characterized in that it is provided with a pressure-sensitive adhesive surface.

3. The hydrogel according to claim 1 or 2 characterized in that active substances contained therein are therapeutic agents to heal skin injuries caused by lesions, UV radiation, aging, or by some other way.

4. The hydrogel according to claim 1 to 3 characterized in that active substances contained therein have a skin-smoothing effect and are selected from the group consisting of vitamins, moisturizers, and vegetable oils.

5. The hydrogels according to claim 1 - 4 characterized in that the active substances contained therein have an effect stimulating the skin function and are selected from the group consisting of plant extracts and essential oils.

6. The hydrogel according to one or several of claims 1 to 5 characterized in that the side averted from the skin is provided with a tack-free layer.

7. The hydrogel according to one or several of claims 1 to 6 characterized in that it is packaged in a waterproof and water vapourproof package prior to application.

8. The use of the hydrogel according to any one of claims 1 to 7 to produce a preparation for healing skin injuries.

9. The use of the hydrogel according to any one of claims 1 to 7 for cosmetic skin care, in particular to smooth and rejuvenate the skin.

10. The use of the hydrogel according to any one of claims 1 to 7 to produce a preparation for influencing the blood circulation of the skin.

11. The use of the hydrogel according to any one of claims 1 to 7 to produce a preparation for the care of the nails.

12. The use of the hydrogel according to any one of claims 1 to 7 for releasing active substances to plants.

## Revendications

1. Hydrogel pour l'application de principes actifs thérapeutiques et/ou cosmétiques sur la peau respectivement pour les soins et le traitement cosmétique d'endroits sensibles de la peau et des ongles, caractérisé en ce qu'il s'agit d'un produit rigide-élastique réalisé sous forme plate et épousant les contours d'endroits du corps humain, qui contient des principes actifs médicaux et/ou cosmétiques.

2. Hydrogel selon la revendication 1, caractérisé en ce qu'il est réalisé avec une surface adhésive.

3. Hydrogel selon la revendication 1 ou 2, caractérisé en ce que les principes actifs qu'il contient sont des agents thérapeutiques pour la guérison de détériorations de la peau provoquées par des blessures, par le rayonnement ultraviolet, par le vieillissement ou encore par d'autres causes.

4. Hydrogel selon `les revendications 1 à 3, caractérisé en ce que les principes actifs qu'il contient ont un effet antirides et sont choisis parmi le groupe constitué par des vitamines, par des agents qui maintiennent humide et par des huiles végétales.

5. Hydrogels selon les revendications 1 à 4, caractérisés en ce que les principes actifs qu'ils contiennent sont des stimulateurs de la fonction cutanée et sont choisis parmi le groupe constitué par des extraits de plantes et par des huiles éthérées.

6. Hydrogel selon une ou plusieurs des revendications 1 à 5, caractérisé en ce qu'il est muni, du côté se détournant de la peau, d'une couche non adhésive.

7. Hydrogel selon une ou plusieurs des revendications 1 à 6, caractérisé en ce qu'il est emballé avant l'application dans un conditionnement étanche à l'eau et à la vapeur d'eau.

8. Utilisation de l'hydrogel selon l'une quelconque des revendications 1 à 7 pour la préparation d'un agent destiné à la guérison de détériorations de la peau.

9. Utilisation de l'hydrogel selon l'une quelconque des revendications 1 à 7 pour des soins cosmétiques de la peau, en particulier pour le déridage, respectivement pour le rajeunissement de la peau.

10. Utilisation de l'hydrogel selon l'une quelconque des revendications 1 à 7 pour la préparation d'un agent destiné à influencer l'irrigation sanguine de la peau.

11. Utilisation de l'hydrogel selon les revendications 1 à 7 pour la préparation d'un agent destiné aux soins des ongles.

12. Utilisation de l'hydrogel selon les revendications 1 à 7 pour distribuer des principes actifs à des plantes.
